# EUROPÄISCHE PATENTSCHRIFT

(11) **EP 1 301 747 B1**
(45) Veröffentlichungstag und Bekanntmachung des Hinweises auf die Patenterteilung: **12.10.2005**
(21) Anmeldenummer: 01953651.5
(22) Anmeldetag: 12.07.2001
(51) Int. Cl.: F23N 5/00, F23G 5/50, G01N 33/28, G01N 33/00

(54) **VERFAHREN ZUR BESTIMMUNG DER RELATION VON NACHWACHSENDEN ZU NICHT NACHWACHSENDEN ENERGIETRÄGERN**
METHOD FOR DETERMINING THE RELATIONSHIP OF RENEWABLE TO NON-RENEWABLE SOURCES OF ENERGY
PROCEDE DE DETERMINATION DE LA RELATION DE PORTEURS D'ENERGIE REGENEREE ET NON REGENEREE

(30) Priorität: 18.07.2000 AT 12542000
(43) Veröffentlichungstag der Anmeldung: 16.04.2003
(73) Patentinhaber: Kneissl, Peter J., 5110 Oberndorf bei Salzburg (AT)
(72) Erfinder: Kneissl, Peter J., 5110 Oberndorf bei Salzburg (AT)
(74) Vertreter: Secklehner, Günter
(86) Internationale Anmeldenummer: PCT/AT2001/000235
(87) Internationale Veröffentlichungsnummer: WO 2002/006730

(56) Entgegenhaltungen:
- WO-A-99/42814
- DE-A- 19 547 258
- AELION C M ET AL: "RADIOCARBON ASSESSMENT OF AEROBIC PETROLEUM BIOREMENDIATION IN THE VADOSE ZONE AND GROUNDWATER AT AN AS/SVE SITE" ENVIRONMENTAL SCIENCE AND TECHNOLOGY, AMERICAN CHEMICAL SOCIETY. EASTON, PA, US, Bd. 31, Nr. 12, 1. Dezember 1997 (1997-12-01), Seiten 3363-3370, XP000751087 ISSN: 0013-936X

## Beschreibung

Die Erfindung betrifft ein Verfahren zur Bestimmung der Relation von nachwachsenden zu nicht nachwachsenden Energieträgern in einem Brennstoffgemisch, insbesondere zur Überwachung der Verbrennung eines Kohlenstoff enthaltenden Brennstoffes sowie eine Vorrichtung zur Durchführung des Verfahrens entsprechend den Merkmalen in den Oberbegriffen der Ansprüche 1 und 14.

Die DE 195 47 258 A offenbart ein Verfahren zum Betrieb einer Müllverbrennungsanlage, welches auf die Messung des stabilen Kohlenstoffisotops C 13 in Form von Kohlendioxid gerichtet ist.

Die Industrialisierung der vergangenen Jahrzehnte führte zu einer Abhängigkeit der Menschheit von sogenannten fossilen Energieträgern. Neben der thermischen Verwertung zur Energiegewinnung werden die Rohstoffe zum Teil auch zur Erzeugung von Produkten benötigt, z.B. Kunststoffen, die aus dem täglichen Leben nicht mehr wegzudenken sind. Der ständig steigende Bedarf von Rohöl, Erdgas, Kohle oder dgl. führte letztendlich zum bekannten Treibhauseffekt, der Schätzungen zufolge zu zwei Dritteln aus Wasserdampf, zu einem Viertel aus CO₂, zu ca. 2 % aus Methan (CH₄) und zu rund 1 % auf andere klimawirksame Atmosphärenbestandteile zurückgeht. Die damit einhergehenden klimatischen Veränderungen veranlassen Regierungen zunehmend, nicht zuletzt aufgrund öffentlichen Druckes, politische Maßnahmen zu setzen, um z.B. die CO₂- bzw. Methan-, FCKW-, Stickoxidemissionen sowie die Emission anderer Spurengase zu verringern. Dies kann einerseits absolut betrachtet dadurch erfolgen, daß aufgrund neuer Technologien eine Reduzierung des Einsatzes der Primärrohstoffe möglich wird. Andererseits ist aber eine relative Verringerung möglich, indem vermehrt erneuerbare Energieträger, wie z.B. Holz, eingesetzt werden. Die Relativabnahme ergibt sich dadurch, daß erneuerbare pflanzliche Energieträger CO₂ im Laufe ihres Lebenszyklus aufnehmen und durch Photosynthese zum Aufbau ihrer Struktur verwenden. Bei der Verbrennung wird also nur jener Teil an CO₂ erzeugt, der ohnehin bereits in der Atmosphäre vorhanden war.

Es ist daher für die Betreiber von Verbrennungsanlagen, z.B. Kraftwerken, von Interesse, die Zusammensetzung der Energieträger in bezug auf das Verhältnis von erneuerbarer zu nicht erneuerbarer Energie zu wissen. Die Ermittlung erfolgte bislang dadurch, daß Proben des Verbrennungsgutes gezogen wurden und diese kosten- und zeitaufwendig analysiert werden mußten.

Aufgabe der vorliegenden Erfindung ist es nun, ein Verfahren sowie eine Vorrichtung anzugeben, mit dem der Zeitaufwand für die Analyse eines Gemisches unterschiedlicher Energieträger verringert werden kann. Es ist weiters eine Teilaufgabe der Erfindung, eine Möglichkeit zur Früherkennung einer Störung einer Verbrennung zu schaffen. Darüber hinaus soll durch die Erfindung der Abfallwirtschaft eine Methode zur Verfügung gestellt werden, mit der eine schnelle Beurteilung der Zusammensetzung eines Gemisches aus unterschiedlichsten Kohlenstoffträgern in Hinblick auf die Methanbildner ermöglicht wird.

Diese Aufgabe wird jeweils eigenständig durch die Merkmale in den Kennzeichenteilen der Ansprüche 1 und 14 gelöst. Vorteilhaft ist daran, daß nunmehr ein einfaches Verfahren sowie eine Vorrichtung zur Durchführung dieses Verfahrens zur Verfügung gestellt werden kann, mit dem bzw. der durch Bestimmung eines einzigen Parameters bzw. weniger Parameter auf die Zusammensetzung eines Brennstoffgemisches in bezug auf das Verhältnis von erneuerbaren zu nicht erneuerbaren Energieträgern rückgeschlossen werden kann und somit innerhalb kürzester Zeit bei Störung dieses Verhältnisses bei der Verwertung der Brennstoffe durch Veränderung des Verhältnisses reagiert werden kann. Damit können derartige Anlagen praktisch vollautomatisch den gesetzlichen Bestimmungen entsprechend betrieben werden. Darüber hinaus bietet dieses Verfahren eine große Sicherheit in bezug auf das Ergebnis, da die menschliche Komponente der Analyse praktisch gegen Null geht, ein Umstand, der sich insbesondere bei Bestimmung einer Vielzahl von Parametern zur Verhältnisermittlung aus den Energieträgern vor deren Verwertung durch Fehlervervielfachung negativ auswirkt.

Weitere vorteilhafte Ausgestaltungen des Verfahrens sind in den Ansprüchen 2 bis 13 gekennzeichnet. Vorteilhaft ist daran, daß die Analyse an Elementen vorgenommen wird, welche in der Natur üblicherweise in zumindest zwei Isotopen vorkommen, und die aufgrund ihrer natürlichen Entstehung sowie ihrem Abbau in einem Gleichgewicht vorliegen und dieses Gleichgewicht durch Wegfall einer der Voraussetzungen der Erhaltung des Gleichgewichtes, beispielsweise durch Absterben des Organismus im Fall von Kohlenstoff, oder durch Abtrennung des Systems, beispielsweise im Fall von Tritium, aus dem Einfluß der Umgebung dadurch gestört wird, daß ein Isotop mit einer bestimmten konstanten Größe abgebaut wird.

Es ist weiters von Vorteil, die Analyse bei Verbrennungsanlagen im Reingas durchzuführen, da keine gesonderte weitere Reinigung des Rauchgases zur Schonung der Analyseeinrichtungen stattfinden muß.

Durch die Abzweigung eines bestimmten Volumens sowie die Bestimmung des Gesamtanteils des Elementes bzw. der dieses enthaltenden Verbindungen kann sowohl ein diskontinuierlicher als auch ein kontinuierlicher Betrieb verwirklicht werden.

Es ist weiters von Vorteil, die Analyse durch Auftrennung nach unterschiedlichen Massen der Isotope durchzuführen, da einerseits mit Standardkomponenten, wie sie aus dem Stand der Technik bekannt sind, gearbeitet werden kann und andererseits aufgrund der schnellen und einfachen Methode die Datensicherheit sehr groß ist bzw. eine Mehrfachanalyse simultan bzw. quasisimultan in einem einzigen Verfahrensschritt durchgeführt werden kann.

Es ist schließlich von Vorteil, das Verfahren dahingehend anzuwenden, daß die Veränderung der Zusammensetzung des Brennstoffgemisches so geändert wird, daß ein definierter Wert für den Anteil an erneuerbaren Energieträgern im Brennstoffgemisch nicht unterschritten wird und diese Änderung durch Zufuhr von erneuerbaren Energieträgern vollautomatisch durchgeführt wird.

In den Ansprüchen 15 bis 19 sind vorteilhafte Ausgestaltungen der erfindungsgemäßen Vorrichtung gekennzeichnet. Von Vorteil ist dabei einerseits, daß die Vorrichtung derart gestaltet werden kann, daß sie in ihrem Aufbau relativ einfach ist, sodaß ein geringer Wartungsaufwand sowie eine hohe Betriebssicherheit erzielt werden kann. Außerdem ist durch die Verbindung der Analyseeinrichtung mit einer Regel- und/oder Steuereinrichtung ein automatischer Betrieb der Vorrichtung erzielbar und kann schließlich eine Verfälschung des Analyseergebnisses durch unvollständig umgesetzte Reaktionsprodukte vermieden werden.

Zum besseren Verständnis wird das Wesen der Erfindung anhand der nachfolgenden Ausführungsbeispiele näher erläutert.

Den wissenschaftlichen Hintergrund für das erfindungsgemäße Verfahren zur Bestimmung des Verhältnisses von erneuerbaren zu nicht erneuerbaren Energieträgern bzw. zur Bestimmung von Abbauprodukten aus Gärungsprozessen oder aus dem anaeroben Abbau stellt die Erkenntnis dar, daß mit Hilfe nicht stabiler Isotope eines Elementes die Bestimmung des Alters des dieses Element enthaltenden Stoffes möglich ist. Eines der bekanntesten Verfahren zur Altersbestimmung stellt die sogenannte Radiokarbon-Methode dar, wie sie z.B. in der Dendrochronologie oder aber auch zur Bestimmung des Alters von Gesteinen, Fossilien oder dgl. verwendet wird. Da diese Methode bestens bekannt ist, soll hier nur ein kurzer Abriß gegeben werden.

Isotope sind Nuklide gleicher Kernladungs- und Ordnungszahl, aber unterschiedlicher Anzahl der im Kern enthaltenen Neutronen und damit unterschiedlicher Massenzahl. Bei der Radiokarbon-Methode wird nun das Verhältnis zweier Isotope, nämlich des stabilen C12 zum radioaktiven C14 benutzt, um das Alter zu bestimmen. C14 wird in der Natur durch Einwirkung kosmischer Strahlung gebildet, indem durch die Höhenstrahlung, d.h. durch ein Neutron ein Stickstoffkern durch Abgabe eines Protons in C14 umgewandelt wird. Aufgrund des radioaktiven Zerfalls von C 14 mit einer Halbwertszeit, welche mit ca. 5.730 Jahren angegeben wird - in der Literatur finden sich auch andere Werte für die Halbwertszeit, jedoch spielen die imaginalen Unterschiede für das Wesen der Erfindung keine bedeutende Rolle - zerfällt dieses C14-Atom wiederum unter Aussendung von β-Strahlen geringer Energie in "normalen Stickstoff".

Frisch gebildete C14-Atome werden in der Erdatmosphäre rasch zu CO₂ oxidiert, das sich gleichmäßig mit dem atmosphärischen "normalen" CO₂ aus C12-Atomen vermischt und zusammen mit diesen in den Kohlenstoffkreislauf (Photosynthese-Nahrungskette-Atmung-Verwesung) eingeht. Damit nehmen lebende Organismen also radioaktive C14-Atome auf und scheiden sie über die Atmung wieder aus. Es bildet sich also ein Gleichgewicht zwischen in Organismen enthaltenen C14-Atomen und wieder ausgeschiedenen C14-Atomen bzw. aufgenommenen C14-Atomen. Der Tod eines Organismus setzt diesem Gleichgewicht ein Ende. Infolgedessen nimmt der C14-Anteil im Organismus mit oben angegebener Halbwertszeit ständig ab. Mit der Halbwertszeit sinkt die Anzahl der β-Zerfälle von anfänglich 16/g C Min. auf 8/g C Min. nach 5760 Jahren, auf 4/g C Min. nach 11520 Jahren, usw. Es besteht also ein Zusammenhang zwischen der verstrichenen Zeit ab dem Absterben des Organismus und der Anzahl der noch vorhandenen C14-Atome infolge der Halbwertszeit. Aus diesem Zusammenhang läßt sich letztendlich das Alter eines Kohlenstoff enthaltenden Organismus bestimmen.

Die Erfindung macht sich diese Erkenntnis insofern zunutze, als daß mit der Radiokarbon-Methode letztendlich eine Bestimmung des Alters bis zu ca. maximal 100.000 Jahre möglich erscheint. Üblicherweise wird dieses Verfahren heute für Zeiträume zwischen 1.000 und 40.000 Jahren angewandt. 100.000 Jahre entsprechen ca. 18 Halbwertszeiten, wobei der Anteil an C14 auf ca. 0,0004 % fällt. Fossile Energieträger sind üblicherweise bedeutend älter als 100.000 Jahre, woraus resultiert, daß der C14-Anteil verschwindend klein bis nicht bestimmbar ist. Erneuerbare kohlenstoffhältige Energieträger, z.B. jede Art von Holz, weisen hingegen ein Alter von weniger als 100.000 Jahren auf und damit einen meßbaren Anteil an C14 Atomen. Wenn nun Gemische unterschiedlichster Energieträger, wie z.B. Gemische aus nicht erneuerbaren und erneuerbaren kohlenstoffhältigen Energieträgern verbrannt werden, so kann durch Bestimmung des C14-Anteils auf das Verhältnis erneuerbar zu nicht erneuerbar rückgeschlossen werden.

Das erfindungsgemäße Verfahren kann aufgrund obiger Gesetzmäßigkeiten zur Bestimmung des Anteils an erneuerbaren Energieträgern bei der thermischen oder chemischen Umsetzung von Abfällen verwendet werden. Dazu kann z.B. in jede beliebige Müllverbrennungsanlage im Rauchgasstrom eine entsprechende Meßstelle angeordnet werden. Diese Meßstelle kann so ausgebildet sein, daß ein bestimmter Anteil des Volumenstromes abgezweigt wird und daraus die Gesamtmenge an CO₂ mit aus dem Stand der Technik bekannten Methoden bestimmt wird. CO₂ wird deshalb bestimmt, da dieses im Regelfall bei der Verbrennung aus kohlenstoffhältigen Quellen gebildet wird. Ein eventueller Anteil an Kohlenmonoxid kann bei den vorherrschenden Temperaturen praktisch mit 0 bemessen werden. Auch bei Vorliegen von Kohle kann bei richtiger Temperaturführung und entsprechender Konzentration an Oxidationsmittel Sauerstoff der Kohlenmonoxidanteil vernachlässigt werden. Sollte Kohlenmonoxid trotzdem in größeren Mengen vorliegen, so besteht die Möglichkeit der Nachbehandlung durch Vervoliständigung der Oxidation, z.B. in einer eigenen Einrichtung wie einem Reaktor, beispielsweise durch Überleiten des Rauchgasstromes über erhitztes CuO. Auf diese Weise können aber auch andere Reaktionsprodukte aus der Verbrennung, z.B. NO, nachträglich vollständig oxidiert werden.

Neben der Bestimmung des Gesamtanteils an CO₂ im Rauchgas erfolgt eine Analyse in bezug auf das Verhältnis von C14/C12 entweder durch Messung von C14 oder durch simultane bzw. quasi simultane Bestimmung sowohl von C14 und C12 und kann dies sowohl kontinuierlich als auch diskontinuierlich durchgeführt werden. Nach dem Vorliegen des Isotopenverhältnisses kann mit einer Mischungsrechnung der Anteil aus biogenem zu fossilem bzw. erneuerbarem zu nicht erneuerbarem Brennstoff über die Halbwertszeit rückgeschlossen werden.

Die Methode ist im wesentlichen unempfindlich gegen Störeinflüsse, welche für die zur Altersbestimmung eingesetzte Radiokarbon-Methode bekannt sind, beispielsweise die Störung des Gleichgewichtes an C14 zu C12 durch Kernwaffenversuche in den letzten Jahrzehnten bzw. andere geogeschichtliche Störeinflüsse. Dies deshalb, da es praktisch nur Extremas gibt, d.h. maximalen C14-Anteil für junge biogene Stoffe wie Holz, Papier oder dgl., bzw. minimalen, gegen Null gehenden C14-Anteil für alte fossile Brennstoffe, wie oben ausgeführt wurde.

Es ist bekannt, daß das Isotopenverhältnis C14 zu C12 im Gleichgewicht 10⁻¹² beträgt. D.h. dieser Wert wird in der Analyse erreicht, wenn 100 % biogener Brennstoff eingesetzt wird. Bei einem 50%igen Anteil verringert sich das Verhältnis entsprechend auf 5 x 10⁻¹³, bei einem 10%igen Anteil auf 1 x 10⁻¹³ usw. D.h. durch Bestimmung dieses Verhältnisses kann direkt auf den prozentuellen Anteil erneuerbarer Energieträger in einem Brennstoffgemisch geschlossen werden. Dies kann entweder rechnerunterstützt automatisch erfolgen, oder aber auch visuell über Meßkurven, in denen das Isotopenverhältnis graphisch in bezug auf prozentuellen Anteil erneuerbarer Energieträger aufgetragen ist.

Wie bereits erwähnt, ist das erfindungsgemäße Verfahren z.B. für Verbrennungsanlagen, insbesondere Müllverbrennungsanlagen, anwendbar. Bekanntlich werden derartige Anlage mit einem Gemisch unterschiedlichster Energieträger, d.h. Kohlenstoffquellen, welche z.B. aus Hausmüll, Industriemüll, Faulschlamm etc. aber auch durch Einsatz von Erdöl, Erdgas, Kohle oder dgl. stammen können, betrieben. Derartige Anlagen sind aus dem Stand der Technik bestens bekannt. Das aus dem Kessel entweichende Rauchgas trägt eine Vielzahl unterschiedlichster Schadstoffe mit sich, sodaß in diesen Anlagen Vorkehrungen zur Rauchgasreinigung getroffen werden. Beispielsweise können Filter wie Elektrofilter zur Entstaubung, Entschwefelungsanlagen in Form von Wäschern, Dioxinfilter, Aktivkohlefiltern, diverse Reinigungen unter Verwendung von Katalysatoren, z.B. zur Stickoxidreduzierung dem Ofen selbst nachgeschaltet sein, bevor das gereinigte Rauchgas als sogenanntes Reingas die Anlage über den Schlot verläßt. Zur Schonung der Analysegeräte bzw. -einrichtungen erfolgt die Messung bevorzugt im Reingas. Dies ist zudem der meist vorgeschriebene Ort zur Abgaskontrolle, da letztendlich das Reingas ohne weitere Behandlung die Verbrennungsanlage verläßt. Vorzugsweise wird der Meßpunkt bzw. die Probenentnahme bei Vorhandensein von Aktivkohlefiltern vor diesen angeordnet, da ansonsten ein Störanteil aus dem Filter mitberücksichtigt werden muß. Letzteres stellt aber bei rechnerunterstützten Systemen kein besonderes Problem dar. Generell kann natürlich auch eine Kalibrierung der Analyseeinrichtungen erfolgen, wobei dies bei dem erfindungsgemäßen Verfahren nicht zwangsweise erforderlich ist. Selbstverständlich können auch entsprechende Eichkurven erstellt werden, indem bestimmte unterschiedliche Mengenverhältnisse an Energieträgern in derartigen Anlagen umgesetzt und die daraus resultierenden Reaktionsprodukte gemessen werden.

Ein weiterer Vorteil, der sich aufgrund der Messung im Reingas ergibt ist, daß an dieser Stelle entsprechende Probennahmeeinrichtungen bereits installiert sind, sodaß auch die Nachrüstung bereits bestehender Anlagen mit entsprechenden Analysatoren zur Durchführung des erfindungsgemäßen Verfahrens problemlos möglich ist. Außerdem sind im Reingasstrom die notwendigen Umrechnungseinrichtungen auf Normalbedingungen des Rauchgases (trocken, feucht, Temperatur, Luftdruck etc.) installiert. Weiters ist auch in der Prozeßüberwachung eine Rauchgasmengenmessung immer installiert und natürlich wird die zugeführte Brennstoffmenge protokolliert, beispielsweise in t/h, kg/h, m³/h etc., wobei u.a. auch auf die Zusammensetzung der Brennstoffe in bezug auf Abfallsekundärbrennstoffe, fossile Brennstoffe etc. aufgrund der Wägeprotokolle bzw. Meßprotokolle rückgeschlossen werden kann.

Mit Hilfe dieser Protokolle kann auf den Summen-CO₂-Gehalt auch über den Anlagenwirkungsgrad rein rechnerisch rückgeschlossen werden, sodaß eine Bestimmung des CO₂-Gehaltes als Summenparameter gegebenenfalls entfallen kann.

Gerade bei Müllverbrennungsanlagen ist es jedoch nahezu unmöglich bzw. nur mit einem sehr hohen zeitlichen und kostenintensiven Aufwand möglich, das Brennstoffgemisch vor der Verbrennung auf Einzelkomponenten zu analysieren. Hier bietet das erfindungsgemäße Verfahren den Vorteil, mit einer raschen Methode aus dem Reingas feststellen zu können, wie hoch der erneuerbare Energieanteil ist. Gesetz den Fall, daß während des Betriebes festgestellt wird, daß sich das Verhältnis erneuerbarer zu nicht erneuerbarer Energieträger zu Ungunsten der erneuerbaren verschiebt, kann eine entsprechende Zudosierung an erneuerbaren Energieträgern, z.B. Holz oder Papier, welche für diesen Zweck gesondert vorrätig gehalten werden können, erfolgen. Dies ist z.B. durch manuelle Zudosierung möglich, vorteilhafterweise wird aber automatisch aufgrund des ermittelten Verhältnisses zudosiert und können dazu entsprechende Regel- und/oder Steuereinrichtungen in der Anlage vorgesehen sein, die mit der Analyseeinrichtung in Datenaustausch stehen.

Zur Bestimmung und Detektion der Kohlenstoffisotope stehen verschiedenste Möglichkeiten zur Verfügung. Einerseits besteht die Möglichkeit eines indirekten Nachweises, in dem CO₂ in Methan oder Ethan umgewandelt wird und die Detektion über Proportionalzählrohre erfolgt. Ebenso kann CO₂ zu Benzol umgesetzt werden und mittels Flüssigkeitszählrohren detektiert werden. Diese Methoden erfordern jedoch eine relativ große Probenmenge von ca. 3 g und sind darüber hinaus zeitaufwendiger.

Zum direkten Nachweis werden vorzugsweise Massenspektrometer, wie z.B. Flugzeit-Massenspektrometer, Massenspektrometer, die vorteilhafterweise mit Tandembeschleunigern verbunden sind, verwendet, in denen eine Trennung von Ionen unterschiedlicher Masse durch Kombination elektrischer und magnetischer Felder erfolgt. Dazu werden in einer Sputterquelle durch Oberflächenionisation erzeugte Cäsiumionen auf die zu untersuchende Probe geschossen. Die dabei entstehenden negativen Ionen des Probenmaterials werden, da die Quelle auf einem negativen Potential liegt, von dieser wegbeschleunigt. In einem 90°-Analysiermagneten erfolgt eine Auftrennung der verschiedenen Isotope nach ihrer Masse und Ladung, wobei um eine quasi Simultanmessung der verschiedenen gesuchten Kohlenstoffisotope durchführen zu können, an die elektrische isolierte Vakuumkammer des Magneten gepulst eine Hochspannung angelegt ist. Dadurch erfahren die Ionen eine zeitweise Energieänderung, sodaß es möglich ist, zwischen den verschiedenen Isotopen umzuschalten. Die in den anschließenden Tandembeschleuniger eintretenden negativ geladenen Ionen werden durch eine positive Spannung zur Tandemmitte hin beschleunigt und treffen dort auf eine Kohlenstofffolie, wodurch durch teilweises Abstreifen der Hüllelektronen eine Umladung erfolgt. Molekulare Teilchen brechen dabei auf und werden später aus dem Ionenstrahl gelenkt. Die verbliebenen Kohlenstoffionen werden von der Tandemmitte wegbeschleunigt und treten beispielsweise unter zweifacher Beschleunigung aus dem Beschleuniger aus. In einem nachfolgenden elektrostatischen Sektorenfeld werden die lonen nach ihrem Ladungszustand ausgewählt sowie im Tandembeschleuniger aufgebrochene molekulare Teilchen abgetrennt. In den folgenden 55° bzw. 120°-Analysiermagneten werden die Teilchen wiederum nach Ihren Massen getrennt, sodaß letztendlich in einem Gasionisationsdetektor die Bestimmung von C 14 erfolgt. Das Isotop C12 sowie das ebenfalls stabile Isotop C13 werden in sogenannten Faradaycups bestimmt, da diese aufgrund des gepulsten Betriebes des 90°-Magneten nach der Sputterquelle kurzzeitig in die Strahlrohrstrecke mit eingeschossen werden. Aus den erhaltenen Daten kann direkt das Verhältnis von C14 zu C12 bestimmt werden und als Grundlage für die weiteren Regelmechanismen in bezug auf Brennstoffeinsatz herangezogen werden.

Neben der beschriebenen Ionisierung durch Cäsiumionen im Massenspektrometer stehen hierfür auch eine Anzahl weiterer Methoden, wie z.B. mittels Laser oder dgl., zur Verfügung.

Daneben bestehen eine Reihe weiterer Möglichkeiten, um C(14)O₂ von C(12)O₂ abzutrennen. So ist es z.B. möglich, mittels Diffusionsverfahren in einer Trennkaskade die beiden Isotope aufgrund unterschiedlicher Diffusionsgeschwindigkeit durch eine semipermeable Wand zu trennen. Die dabei nicht diffundierte schwerere Fraktion wird an die jeweils vorhergehenden Stufe zurückgeführt. Die Diffusion kann durch einen Druckunterschied zwischen Innen und Außen je Trennstufe beschleunigt werden, wobei die Druckdifferenz je nach Membran zwischen 10 und 100 mbar betragen kann.

Eine Trennung nach der Ultrazentifugentechnik ist ebenfalls möglich, wobei die Isotopen aufgrund ihrer unterschiedlichen Massen auf unterschiedliche Kreisbahnen durch Beschleunigung gelenkt werden und somit getrennt abgezogen werden können.

In Weiterbildung dieses Verfahrens können beim sogenannten Plasmaverfahren in einem unter der Wirkung magnetischer Felder rotierenden Plasma wesentlich höhere Umlaufgeschwindigkeiten als bei einer Zentrifuge erreicht werden und ist somit eine Schwerkrafttrennung möglich. Dabei wird zusätzlich als zweiter Effekt ausgenutzt, daß in einem gleichförmigen Magnetfeld die Zyklotronfrequenz der Ionen im Plasma nur von der Magnetfeldstärke und der Ionenmasse abhängt. Dadurch bilden sich im Zyklotron unterschiedliche Umlaufbahnen der Isotope aus.

Es sei an dieser Stelle vermerkt, daß die beschriebenen Methoden selbstverständlich nur stellvertretend für jedwede anderen, aus dem Stand der Technik bekannten Analysemethoden, die zur Trennung unterschiedlicher Isotope bzw. zur Bestimmung eines Isotopenverhältnisses geeignet sind, stehen, sodaß das erfindungsgemäße Verfahren nicht auf die angegebenen analytischen Methoden beschränkt ist.

Anstelle der Messung im Reingas ist es selbstverständlich möglich, bereits zu einem früheren Zeitpunkt, z.B. nach der Entstaubung eine Analyse des Rauchgases in bezug auf das Isotopenverhältnis C14/C12 vorzunehmen, wobei gegebenenfalls eine gesonderte Rauchgasreinigung für die abgezweigten, zu analysierenden Mengen vorgeschaltet werden kann. Darüber hinaus ist es damit ebenfalls möglich, ohne weitere Reinigungsstufen eine Gesamtanalyse der Rauchgasinhaltsstoffe, z.B. auf SO₂, auf Stickoxide oder dgl. vorzunehmen, sodaß letztendlich nur eine einzige Probenentnahme in der Gesamtanlage erfolgen muß.

Für das erfindungsgemäße Verfahren gibt es eine Reihe weitere Anwendungsmöglichkeiten, z.B. den Einsatz in einer industriellen Mitverbrennungsanlage. Im wesentlichen unterscheidet sich dieses Ausführungsbeispiel nicht vom vorhergehend beschriebenen, mit der Ausnahme, daß derartige Anlagen nicht zwangsweise auf Müllentsorgung ausgerichtet sind und unter Umständen eine Rückgewinnung von Sekundärrohstoffen aus den Verbrennungsprodukten im Vordergrund stehen kann. Die Messung des Verhältnisses C14/C12 erfolgt wiederum bevorzugt im Reingas.

In einer weiteren Variante kann über den prozentuellen Anteil an C14-Atomen, z.B. aus dem Deponiegas, auf die Zusammensetzung des Deponiegutes rückgeschlossen werden. In diesem Fall steht naturgemäß nicht ausschließlich die CO₂-Bestimmung im Vordergrund, sondern kann der Anteil an erneuerbaren Energieträgern über Methan bestimmt werden, welches als sogenanntes Deponiegas entweicht. Vorzugsweise findet die Bestimmung wieder nach einer vorgeschalteten Reinigung, z.B. einer Staubabscheidung durch eine entsprechende Filteranlage oder dgl. statt.

Als Variante dazu ist es möglich, anstelle der Bestimmung über Methan wiederum den CO₂-Weg einzuschlagen, indem das Deponiegas für Gasmotoren, welche beispielsweise zur Stromerzeugung eingesetzt werden, verwendet wird und im Gasmotor zu CO₂ verbrannt wird. Gegebenenfalls kann es hier günstig sein, den Kohlenmonoxidanteil mitzubestimmen.

Schließlich ist es möglich, auch im unverbrannten Rückstand eine Bewertung des Verhältnisses erneuerbarer zu nicht erneuerbarer Energieform vorzunehmen, indem mit dem erfindungsgemäßen Verfahren die Restprodukte, wie Schlacke, Asche oder dgl. untersucht werden.

Anstelle der Methanbestimmung aus dem Deponiegas kann selbstverständlich auch das beim oxidativen Abbau entstehende CO₂ der Deponie zur Bestimmung der Zusammensetzung der Deponie herangezogen werden.

Selbstverständlich besteht die Möglichkeit, auch andere Radio-Isotope für das erfindungsgemäße Verfahren zu verwenden, wobei gewisse Bedingungen erfüllt sein müssen. Entweder müssen die entsprechenden Isotope, wie im Fall des C14, ständig neu ergänzt werden, oder muß generell die Halbwertszeit so bemessen sein, daß ein Anteil des Radioisotopes in fossilen Energieträgern nicht mehr nachgewiesen werden kann.

Eines dieser Isotope ist z.B. Tritium. Tritium entsteht in der Erdatmosphäre auf ähnliche Weise wie C14 aus Stickstoff durch Neutronenbeschuß unter gleichzeitiger Bildung von C12. Daneben entsteht es aus Stickstoff durch Reaktion mit Wasserstoff bzw. durch Reaktion von Deuteriumatomen miteinander. Aufgrund der sehr kurzen Halbwertszeit von ca. 12,3 Jahren eignet sich Tritium lediglich zur Bestimmung sehr junger erneuerbarer Energieträger. Bei der Verbrennung entsteht durch Oxidation mit Sauerstoff Tritiumdioxid, welches beispielsweise ebenfalls mittels Isotopentrennung vom normalen Wasserdampf abgetrennt werden kann.

Tritium bietet aber auch eine interessante Variante des erfindungsgemäßen Verfahrens, da bereits in der Atmosphäre Tritiumdioxid gebildet wird. Dieses beteiligt sich am normalen Wasserkreislauf, sodaß also in Systemen, welche nach einiger Zeit vom natürlichen Wasserkreislauf abgetrennt werden, Tritium mit genannter Halbwertszeit als ebenfalls schwacher β-Strahler radioaktiv zerfällt. D.h. man kann z.B. bei Deponiewässern eine Unterscheidung treffen, ob das in der Deponie enthaltene Wasser aus Oberflächenwässern oder aus Grundwasser stammt, sodaß auf Undichtigkeiten der Deponie rückgeschlossen werden kann.

Es sei vermerkt, daß es aufgrund des Einwirkens des Menschens auf die Umwelt mittlerweile auch möglich ist, Isotope von radioaktiven Elementen, z.B. Plutonium, unter Umständen für das erfindungsgemäße Verfahren heranzuziehen, da diese über Kernwaffentests oder auch die zivile Kernenergienutzung und die daraus resultierenden Störfälle in die Umwelt gelangten und somit insbesondere von Bäumen oder auch anderen Pflanzenarten während ihres Wachstums aufgenommen worden sind. Deren Einsetzbarkeit kann aber regional beschränkt sein und wird die Kenntnis der Vorgeschichte dieser Pflanzen vorausgesetzt.

Neben der Bestimmung der Relation von Energieträgern zueinander kann das erfindungsgemäße Verfahren prinzipiell auch zur Überwachung des Verbrennungsprozesses herangezogen werden. Wird nämlich festgestellt, daß z.B. der CO₂-Anteil im Rauchgas unerwartet fällt, so deutet dies auf einen Störfall infolge unvollständiger Verbrennung hin.

Abschließend sei erwähnt, daß sich das erfindungsgemäße Verfahren nicht ausschließlich auf die genannten Ausführungsvarianten bezieht, sondern der Fachmann aufgrund seines Wissens durch obenstehende Leere entsprechende Varianten, insbesondere in Hinblick auf Isotope bzw. Bestimmungsmethoden der Isotope auffinden kann und sind diese vom Schutzumfang mit umfaßt.

## Patentansprüche

1. Verfahren zur Bestimmung der Relation von nachwachsenden zu nicht nachwachsenden Energieträgern in einem Brennstoffgemisch, insbesondere für Verbrennungsanlagen oder Mülldeponien, bei dem aus den Energieträgern zumindest ein gasförmiges und/oder festes und/oder flüssiges Reaktionsprodukt erzeugt und analysiert wird, **dadurch gekennzeichnet, daß** der Anteil zumindest eines bereits im Brennstoffgemisch vorhandenen instabilen, radioaktiven Elementes und/oder zumindest einer dieses enthaltenden Verbindung am Reaktionsprodukt bestimmt wird.

2. Verfahren nach Anspruch 1, **dadurch gekennzeichnet, daß** ein Element für die Analyse ausgewählt wird, welches im Brennstoffgemisch mit zumindest zwei unterschiedlichen Isotopen enthalten ist.

3. Verfahren nach Anspruch 1 oder 2, **dadurch gekennzeichnet, daß** das Element Kohlenstoff oder Wasserstoff bzw. Tritium ist.

4. Verfahren nach einem der vorhergehenden Ansprüche, **dadurch gekennzeichnet, daß** die Verbindung CO₂ und/oder Wasser und/oder CH₄ ist.

5. Verfahren nach Anspruch 3 oder 4, **dadurch gekennzeichnet, daß** das Verhältnis von ¹²C zu ¹⁴C und/oder ¹H zu ³H bestimmt wird.

6. Verfahren nach einem der vorhergehenden Ansprüche, **dadurch gekennzeichnet, daß** zur Analyse ein bestimmtes Volumen oder eine bestimmte Menge eines aus der Oxidation, insbesondere der Verbrennung, entstandenen Rauchgases abgezweigt und einer Analyseeinrichtung zugeführt wird.

7. Verfahren nach einem der vorhergehenden Ansprüche, **dadurch gekennzeichnet, daß** der Gesamtanteil des Elementes bzw. der Verbindung am abgezweigten Volumen bestimmt wird.

8. Verfahren nach einem der vorhergehenden Ansprüche, **dadurch gekennzeichnet, daß** die Analyse in einem aus dem Rauchgas hergestellten Reingas durchgeführt wird.

9. Verfahren nach einem der vorhergehenden Ansprüche, **dadurch gekennzeichnet, daß** die Analyse an einem bestimmten Volumen eines Deponiegases und/oder eines Reaktionsproduktes daraus, z.B. CO₂, durchgeführt wird.

10. Verfahren nach einem der vorhergehenden Ansprüche, **dadurch gekennzeichnet, daß** zur Analyse eine Auftrennung nach den unterschiedlichen Massen der Isotope durchgeführt wird.

11. Verfahren nach Anspruch 10, **dadurch gekennzeichnet, daß** die Auftrennung mit einem Massenspektrometer (MS), z.B. einem Flugzeit-MS, einem MS mit Tandembeschleuniger und/oder einer Trennkaskade aufgrund unterschiedlichem Diffusionsverhalten und/oder einer Ultrazentrifuge und/oder nach einem Plasmaverfahren erfolgt.

12. Verfahren nach einem der Ansprüche 6 bis 11, **dadurch gekennzeichnet, daß** nach Feststellung des Anteils an dem instabilen Isotop im Rauch- bzw. Reingas in Anhängigkeit eines vordefinierbaren Wertes für den Anteil an erneuerbaren Energieträgern im Brennstoffgemisch die eingesetzte Menge an dem bzw. den erneuerbaren Energieträger(n) verändert wird.

13. Verfahren nach Anspruch 12, **dadurch gekennzeichnet, daß** die Veränderung der Zusammensetzung des Brennstoffgemisches automatisch durch eine Regel- und/oder Steuereinrichtung, die mit der Analyseeinrichtung in Datenverbindung steht, veranlaßt wird.

14. Vorrichtung zur Durchführung des Verfahrens nach einem der Ansprüche 1 bis 13, mit einer Verbrennungseinrichtung, z.B. einem Ofen, einem Motor, insbesondere geeignet zur thermischen Verwertung von Abfallstoffen, mit zumindest einer Reinigungseinrichtung für das aus der Verbrennung stammende Rauchgas, z.B. einem Filter, einem Rauchgaswäscher für SO₂, Stickoxide, sowie mit einer Rauchgasleitung, die in einen Schlot mündet und in der eine Entnahmestelle angeordnet ist, über die ein bestimmtes Volumen bzw. eine bestimmte Menge des Rauchgases aus dem Rauchgasstrom abgezweigt werden kann, **dadurch gekennzeichnet, daß** die Entnahmestelle mit einer Analyseeinrichtung zur Bestimmung eines instabilen, radioaktiven Isotops eines Elementes bzw. eines Isotopenverhältnisses und/oder zur Bestimmung von aus dem Element bzw. den Isotopen gebildeten Reaktionsprodukten aus der Verbrennung, gegebenenfalls unter Zwischenschaltung zumindest einer weiteren Reinigungsstufe, verbunden ist.

15. Vorrichtung nach Anspruch 14, **dadurch gekennzeichnet, daß** die Analyseeinrichtung zur Auftrennung der Reaktionsprodukte aus der Verbrennung nach ihrer Masse ausgebildet ist.

16. Vorrichtung nach Anspruch 15, **dadurch gekennzeichnet, daß** die Analyseeinrichtung ein Massenspektrometer, gegebenenfalls mit Tandembeschleuniger ist.

17. Vorrichtung nach einem der Ansprüche 14 bis 16, **dadurch gekennzeichnet, daß** die Entnahmestelle in dem Teil der Rauchgasleitung angeordnet ist, der bereits Reingas führt,

18. Vorrichtung nach einem der Ansprüche 14 bis 17, **dadurch gekennzeichnet, daß** die Analyseeinrichtung mit einer Regel- und/oder Steuereinrichtung verbunden ist, über die die Zusammensetzung der dem Verbrennungsofen zugeführten Brennstoffe geregelt wird.

19. Vorrichtung nach einem der Ansprüche 14 bis 18, **dadurch gekennzeichnet, daß** vor der Analyseeinrichtung eine Einrichtung, z.B. ein Reaktor, angeordnet ist, in der bei Vorliegen einer unvollständig umgesetzten Verbindung, z.B. CO, aus der Verbrennung oder der Gassammelstelle eine Nachreaktion der Verbindung durchgeführt werden kann, damit das zu bestimmende Isotop in der höchsten Oxidationsstufe vorliegt.

## Claims

1. Method of determining the relationship of renewable to non-renewable sources of energy in a fuel mixture, in particular for incineration plants or refuse deposits, whereby at least a gaseous and/or solid and/or liquid reaction product is generated from the energy sources and analysed, **characterised in that** the proportion of at least one unstable, radioactive element already present in the fuel mixture and/or at least a compound containing it is determined on the basis of the reaction product.

2. Method as claimed in claim 1, **characterised in that** an element which is contained in the fuel mixture with at least two different isotopes is selected for the analysis.

3. Method as claimed in claim 1 or 2, **characterised in that** the element is carbon or hydrogen or tritium.

4. Method as claimed in one of the preceding claims, **characterised in that** the compound is CO₂ and/or water and/or CH₄.

5. Method as claimed in claim 3 or 4, **characterised in that** the ratio of ¹²C to ¹⁴C and/or ¹H to ³H is determined.

6. Method as claimed in one of the preceding claims, **characterised in that** a specific volume or a specific quantity of a flue gas resulting from oxidation, in particular from combustion, is branched off for analysis purposes and delivered to an analysis unit.

7. Method as claimed in one of the preceding claims, **characterised in that** the total proportion of the element or the compound is determined on the basis of the branched-off volume.

8. Method as claimed in one of the preceding claims, **characterised in that** the analysis is conducted in a cleaned gas obtained from the flue gas.

9. Method as claimed in one of the preceding claims, **characterised in that** the analysis is conducted on the basis of a specific volume of a reservoir gas and/or a reaction product obtained from it, e.g. CO₂.

10. Method as claimed in one of the preceding claims, **characterised in that** a separation is undertaken on the basis of the different masses of the isotopes for analysis purposes.

11. Method as claimed in claim 10, **characterised in that** the separation is effected by means of a mass spectrometer (MS), e.g. a Time of Flight MS, a MS with tandem accelerator and/or a separator cascade on the basis of differing diffusion behaviour and/or an ultracentrifuge and/or by a plasma method.

12. Method as claimed in one of claims 6 to 11, **characterised in that**, after determining the proportion on the unstable isotope in the flue gas or cleaned gas depending on a pre-definable value for the proportion of renewable energy sources in the fuel mixture, the quantity of the renewable energy source or sources used is changed

13. Method as claimed in claim 12, **characterised in that** the change in the composition of the fuel mixture is made automatically by means of a regulating and/or control system having a data connection to the analysis unit.

14. Device for implementing the method as claimed in one of claims 1 to 13, with a combustion unit, e.g. an oven, a motor, in particular one which is suitable for conducting a thermal evaluation of waste materials, having at least one cleaning unit for the flue gas emanating from the combustion process, e.g. a filter, a flue gas scrubber for SO₂, nitrogen oxide, as well as a flue gas line opening into a chimney in which a tapping-off point is disposed, by means of which a specific volume or a specific quantity of the flue gas can be drawn off from the flue gas flow, **characterised in that** the tapping-off point is connected to an analysis unit for determining an unstable, radioactive isotope of an element or an isotope ratio and/or for identifying reaction products formed from the element or the isotopes due to combustion, optionally with at least one other scrubbing stage connected in between.

15. Device as claimed in claim 14, **characterised in that** the analysis unit separates the reaction products from the combustion process on the basis of their mass.

16. Device as claimed in claim 15, **characterised in that** the analysis unit is a mass spectrometer, optionally with tandem accelerator.

17. Device as claimed in one of claims 14 to 16, **characterised in that** the tapping-off point is disposed in the part of the flue gas line through which the already cleaned gas is conveyed.

18. Device as claimed in one of claims 14 to 17, **characterised in that** the analysis unit is connected to a regulating and/or control system, by means of which the composition of the fuels delivered to the incinerator oven is automatically regulated.

19. Device as claimed in one of claims 14 to 18, **characterised in that** a unit, e.g. a reactor, is disposed upstream of the analysis unit, in which a compound such as CO, for example, from the combustion process or the gas collection point which has not been fully reacted can be subjected to another reaction so that the isotope to be determined is present in the highest stage of oxidation.

## Revendications

1. Procédé pour la détermination de la relation entre des porteurs d'énergie se régénérant et ne se régénérant pas dans un mélange combustible, en particulier pour des installations de combustion ou des dépôts d'ordures dans lequel, à partir des porteurs d'énergie, au moins un produit réactionnel gazeux et/ou solide et/ou liquide est produit et analysé, **caractérisé en ce que** la proportion d'au moins un élément radioactif instable déjà présent dans le mélange combustible et/ou d'au moins un composé le contenant par rapport au produit réactionnel est déterminée.

2. Procédé selon la revendication 1, **caractérisé en ce qu'**un élément pour l'analyse est choisi, qui est contenu dans le mélange combustible avec au moins deux isotopes différents.

3. Procédé selon la revendication 1 ou 2, **caractérisé en ce que** l'élément est du carbone ou de l'hydrogène ou respectivement du tritium.

4. Procédé selon l'une quelconque des revendications précédentes, **caractérisé en ce que** le composé est CO₂ et/ou de l'eau et/ou CH₄.

5. Procédé selon la revendication 3 ou 4, **caractérisé en ce que** le rapport de ¹²C à ¹⁴C et/ou ¹H à ³H est déterminé.

6. Procédé selon l'une quelconque des revendications précédentes, **caractérisé en ce que**, pour l'analyse d'un volume déterminé et/ou d'une quantité déterminée d'un gaz de fumée résultant de l'oxydation, en particulier de la combustion, est détourné et conduit à un dispositif d'analyse.

7. Procédé selon l'une quelconque des revendications précédentes, **caractérisé en ce que** la quantité totale de l'élément ou respectivement du composé est déterminé sur le volume détourné.

8. Procédé selon l'une quelconque des revendications précédentes, **caractérisé en ce que** l'analyse est accomplie dans un gaz pur produit à partir des gaz de fumée.

9. Procédé selon l'une quelconque des revendications précédentes, **caractérisé en ce que** l'analyse est accomplie sur un volume déterminé d'un gaz de dépôt et/ou d'un produit réactionnel qui en est formé, par exemple CO₂.

10. Procédé selon l'une quelconque des revendications précédentes, **caractérisé en ce que**, pour l'analyse, une séparation est effectuée selon les différentes masses des l'isotopes.

11. Procédé selon la revendication 10, **caractérisé en ce que** la séparation se produit avec un spectromètre de masse (SM), par exemple, un SM par temps de vol, un SM avec cascade en tandem d'accélération et/ou séparation sur la base de différents rapports de diffusion et/ou en ultracentrifugeuse et/ou un procédé au plasma.

12. Procédé selon l'une quelconque des revendications 6 à 11, **caractérisé en ce qu'**après avoir établi la proportion de l'isotope instable dans le gaz de fumée ou respectivement propres, par rapport à une valeur prédéfinie pour la proportion de porteurs d'énergie se régénérant dans le mélange combustible, la quantité utilisée de porteur(s) d'énergie pouvant être régénérée est modifiée.

13. Procédé selon la revendication 12, **caractérisé en ce que** la modification de la composition du mélange combustible est effectuée automatiquement par un système de régulation et/ou de commande qui est en liaison de données avec l'appareil d'analyse.

14. Dispositif pour l'accomplissement du procédé selon l'une quelconque des revendications 1 à 13, avec un système de combustion, par exemple un four, un moteur, en particulier approprié pour la mise en valeur thermique de déchets avec au moins un dispositif de nettoyage pour les gaz de fumée provenant de la combustion, par exemple un filtre, un laveur de gaz de fumée pour SO₂, l'oxyde d'azote ainsi qu'une conduite de gaz de fumée qui débouche dans une cheminée et qui se trouve dans un point de prélèvement par lequel peut être dérivé un volume déterminé ou respectivement une quantité déterminée des gaz de fumée du courant de gaz de fumée, **caractérisé en ce que** le point de prélèvement est relié à un système d'analyse pour la détermination d'un isotope instable, radioactif d'un élément ou respectivement d'un rapport d'isotopes et/ou la détermination des produits réactionnels formés de l'élément ou respectivement des isotopes provenant de la combustion, le cas échéant avec incorporation d'au moins un autre étage d'épuration.

15. Dispositif selon la revendication 14, **caractérisé en ce que** le système d'analyse est configuré pour la séparation des produits réactionnels provenant de la combustion selon leur masse.

16. Dispositif selon la revendication 15, **caractérisé en ce que** le système d'analyse est un spectromètre de masse, le cas échant avec accélérateur en tandem.

17. Dispositif selon l'une quelconque des revendications 14 à 16, **caractérisé en ce que** le point de prélèvement est agencé dans la partie de la conduite de gaz de fumée qui conduit déjà le gaz pur.

18. Dispositif selon l'une quelconque des revendications 14 à 17, **caractérisé en ce que** le système d'analyse est relié à un système de régulation et/ou commande par lequel est réglée la composition des gaz de combustion conduits au four de combustion.

19. Dispositif selon l'une quelconque des revendications 14 à 18,**caractérisé en ce que** devant le système d'analyse est agencé un système, par exemple un réacteur, dans lequel, en présence d'un composé qui n'est pas totalement transformé, par exemple CO, provenant de la combustion ou du point de collecte de gaz, une post-réaction du composé peut être accomplie de façon que l'isotope indéterminé se trouve au plus fort état d'oxydation.
